Europäisches Patentamt

European Patent Office

Office européen des brevets

(11) Publication number: 0 430 093 A2

# EUROPEAN PATENT APPLICATION

(21) Application number: 90122391.7

(22) Date of filing: 23.11.90

(51) Int. Cl.5: G01S 15/89, A61B 8/06

(30) Priority: 23.05.90 US 527565
27.11.89 US 441861

(43) Date of publication of application:
05.06.91 Bulletin 91/23

(84) Designated Contracting States:
DE FR GB IT Bulletin

(71) Applicant: ACOUSTIC IMAGING
TECHNOLOGIES CORPORATION
10027 South 51st Street
Phoenix, Arizona 85044(US)

(72) Inventor: Jaeger, Paul M.
8134 E. Willettas
Meza, Arizona 85207(US)
Inventor: Sturgill, Michael R.
3501 E. Morrow Drive
Phoenix, Arizona 85024(US)

(74) Representative: Kasseckert, Rainer
DORNIER GMBH Kleeweg 3
W-7990 Friedrichshafen 1(DE)

(54) Ultrasonic doppler imaging with analog feedback signal canceller.

(57) An ultrasonic blood flow measuring and imaging system comprises a transmit-receive transducer for transmitting ultrasonic waves toward and into the human body and for receiving reflected echo signals which are then processed for use in a Doppler blood flow imaging and display system. Multiple ultrasonic pulse beams are transmitted into the body at each of a number of locations in an area under diagnosis. For each location a plurality of reflected echo signals are received during successive predetermined time intervals. Each received echo signal has a stationary component reflected from essentially stationary tissue and a Doppler component reflected from areas where movement is sensed, such as blood flow. The reflected echo signals are processed in a canceller having stationary signal acquisition and Doppler signal acquisition modes, in that order, for each flow measuring sequence. In the stationary acquisition mode, an ultrasound basis line is first transmitted, its echo detected, amplified, converted to digital, and then stored in a line buffer. The system then switches to the Doppler acquisition mode, and on each of the succeeding pulses the stored line samples are recalled from the line buffer, converted to analog form, and subtracted from the succeeding reflected echo signals. The difference is amplified, converted to digital, and processed by an MTI filter to thereby produce Doppler flow image data for use in imaging, including color imaging, of blood flow in the area under diagnosis.

## ULTRASONIC DOPPLER IMAGING WITH ANALOG FEEDBACK SIGNAL CANCELLER

Cross-Reference

This is a continuation-in-part of our application Serial No. 07/441,861, filed November 27, 1989.

Field of the Invention

This invention relates to ultrasonic diagnosis techniques, and more particularly to ultrasonic Doppler flow imaging and display systems. The invention is particularly useful for improving the dynamic range of a Doppler ultrasound blood flow imaging system.

Background of the Invention

Various techniques have been used in the past to achieve noninvasive imaging of blood flow using ultrasound. Recent developments in Doppler echocardiography are an example. Although the present invention is applicable to other uses, it will be described below in connection with its applicability to Doppler ultrasound blood flow imaging.

A typical ultrasound blood flow imaging system includes an ultrasonic transmit-receive transducer for transmitting ultrasonic pulses into a region of the body under diagnosis and for receiving echo signals of the transmitted ultrasound waves reflected due to blood flow in the area being scanned. A typical diagnosis with ultrasound includes scanning the patient with the ultrasound probe to measure blood flow in an artery, a vein, or in the heart. A signal processing system processes the received echo signals for measuring the Doppler shift frequency of the echo signals to thereby calculate the velocity of the blood flow, and the result of the velocity distribution measurement is displayed as a Doppler blood flow image. Typically, echo amplitudes are displayed in shades of gray (a "B-Mode" display). The Doppler blood flow image is displayed in color, overlaid on the gray scale.

In order to estimate the Doppler shifts of the echoes received from the blood cells, an ultrasound imaging system commonly transmits several (perhaps from 4 to 16) pulses at one location in the region under diagnosis and then detects the variations in the phase of the echoes from pulse to pulse.

Echo signal components Doppler-shifted by the blood flow are extracted from the Doppler signal components carrying the information of the internal moving part of the body. Echo signal components reflected from stationary or slowly moving targets, such as the wall of the heart or blood vessels, whose speed is slower than that of blood, are removed. Those signals are referred to as "clutter" and are removed to extract only the signal components Doppler-shifted by the blood flow being measured.

An MTI (moving target indication) filter (also referred to as a Delay Line Filter (DLF) or a stationary canceller) is used to eliminate clutter signals. In a typical MTI filter, echo signals for consecutive sound receptions are subtracted. Since the echo from blood flow is superimposed on a strong tissue echo, such as that from blood vessels or the heart wall, the subtraction steps can, on the average, eliminate the signal due to stationary or slowly moving objects and extract only the Doppler component representing blood flow. The MTI filter output is then processed to extract the Doppler frequency information, which is converted to velocity data displayed in color, along with a conventional B-mode gray scale display of echo amplitudes.

A principal object in the design and development of a Doppler blood flow imaging system is to improve flow image quality. This can be achieved by signal processing techniques that result in higher Doppler signal-to-clutter ratios. It is also important to provide such improvements without adversely affecting system costs or image frame rates.

Summary of the Invention

The present invention provides an analog feedback stationary signal canceller for ultrasound flow imaging having improved Doppler signal-to-noise ratios. Briefly, one embodiment of the invention includes an ultrasonic wave transmitting and receiving device for sequentially transmitting ultrasonic waves toward and into a living body over a predetermined time interval and for receiving their reflected echo signals. In each flow measuring sequence, a series of ultrasound signals in the form of pulse-echo sequences or data line samples are transmitted-toward and received from a selected location under diagnosis over a predetermined time interval. Each received echo signal has a stationary component representative of reflection from essentially stationary tissue and a Doppler component representative of reflection from a faster moving part of the body such as blood flow. The echo signals received during successive predetermined time intervals are processed by signal processing means including (i) means for subtracting a stationary component from the received echo signals to thereby extract from the echo signals a Doppler signal representative of a Doppler component of the received echo signals, and (ii) means for thereafter amplifying the Doppler signal to produce a succession of output signals representative of the amplified Doppler components of the reflected echo signals. The extracted and amplified Doppler output signals are then processed to generate Doppler flow image data signals for use in imaging the moving part of the body under diagnosis.

In a preferred form of the invention, the reflected echo signals are processed in an analog feedback signal canceller having stationary signal acquisition and Doppler signal acquisition modes. During each flow measuring sequence, the signals from one ultrasound line are converted to digital and stored in a line buffer. The system then switches to the Doppler acquisition mode, and on each of the succeeding pulses during the same flow measuring sequence, the stored line samples are recalled from the line buffer, converted to analog form, and subtracted from the succeeding reflected echo signals. The difference is then amplified, converted to digital, and processed by an MTI filter and velocity estimator to thereby produce Doppler flow image data for use in imaging, including color imaging, of blood flow (velocity) in the area under diagnosis.

One advantage of the invention is that the system produces a higher ratio of Doppler signal-to-quantization noise and permits the use of less expensive analog-to-digital converters, with only a small decrease in image frame rate.

These and other aspects of the invention will be more fully understood by referring to the following detailed description and the accompanying drawings.

Brief Description of the Drawings

FIG. 1 is a schematic functional block diagram illustrating components of a blood flow measuring and imaging system for producing input signals to the systems illustrated in FIGS. 2 and 4;

FIG. 2 is a functional block diagram illustrating a prior art stationary signal canceller for an ultrasonic flow imaging system;

FIG. 3 is a functional block diagram illustrating a conventional use of the information produced by the systems shown in FIGS. 2 and 4 for ultrasonic flow imaging of the received information; and

FIG. 4 is a functional block diagram illustrating an analog feedback stationary signal canceller according to principles of this invention.

Detailed Description

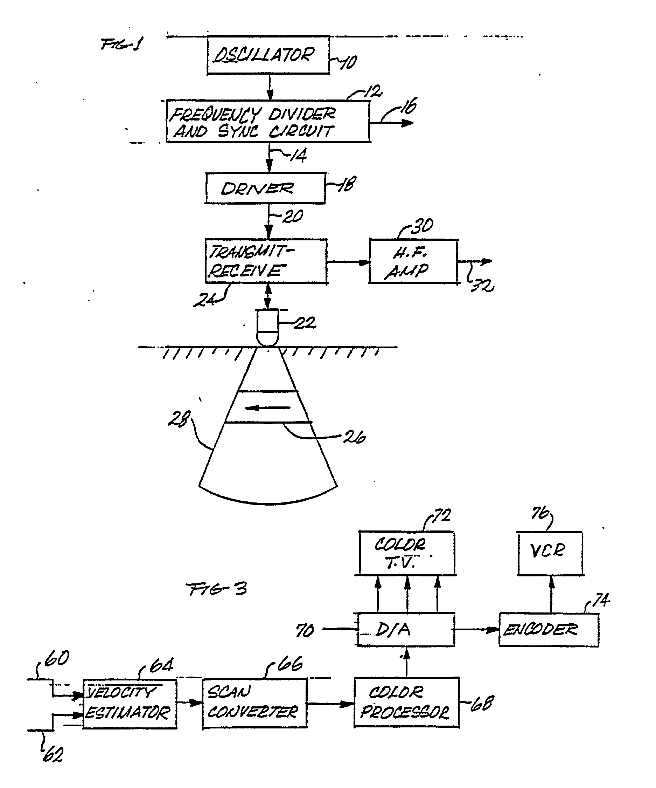

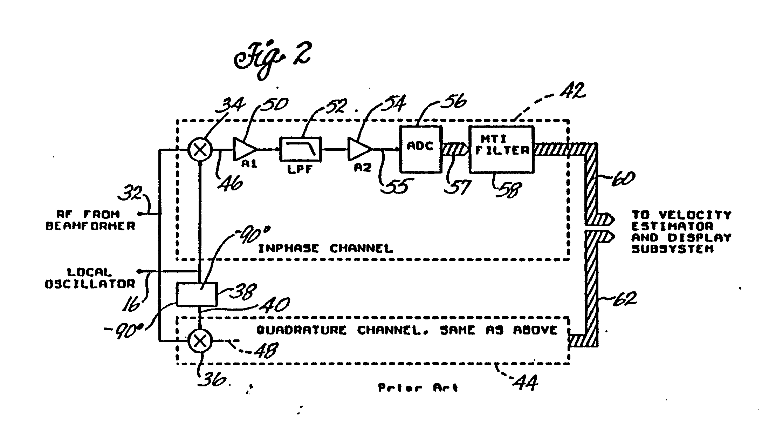

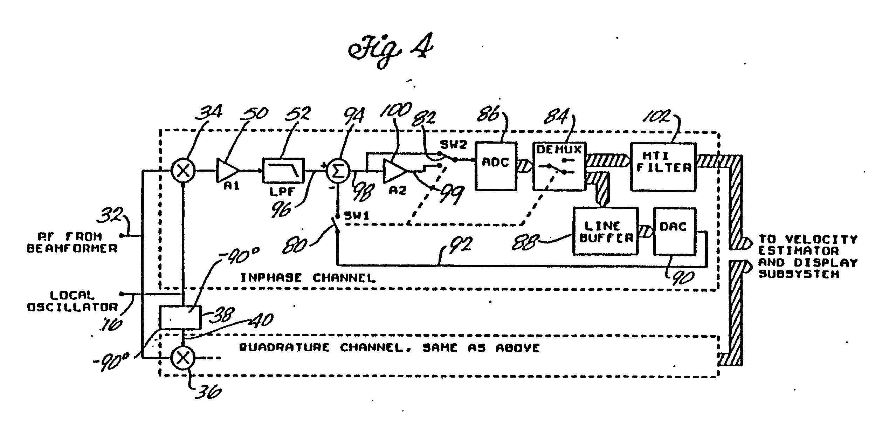

Referring to the drawings, FIGS. 1 and 3 illustrate conventional components of a blood flow measuring and imaging system. FIG. 2 illustrates components of a prior art stationary signal canceller for ultrasonic flow imaging. FIG. 1 is an example of one means for producing input signals to the FIG. 2 system, and FIG. 3 illustrates one type of signal processing and display means for processing and displaying the output of the prior art FIG. 2 system. FIG. 4 illustrates components of an analog feedback stationary signal canceller for ultrasonic flow imaging according to principles of this invention. The system of FIG. 4 can be used with the input derived from the FIG. 1 system and can be used with the signal processing and image display system shown in FIG. 3.

Referring now to FIG. 1, a blood flow measuring system includes an oscillator 10 which generates a stable high frequency oscillation signal applied to a frequency-dividing and synchronizing circuit 12. In response to the high frequency oscillation signal, the circuit 12 generates a digital pulse signal 14 for ultrasonic pulse beam transmission and a signal 16 for quadrature detection. In response to the digital pulse signal 14, a driver circuit 18 applies an analog pulse signal 20 to a probe 22 through a transmit-receive change-over circuit 24. The probe is excited to transmit an ultrasonic pulse beam toward a blood vessel 26 in a sector 28 of a living body under examination. The signal reflected from the blood vessel 26 is converted by the probe 22 into an electrical signal, and this signal is applied, through the transmit-receive change-over circuit 24, to a high frequency amplifier 30. An amplified output signal 32 is applied to quadrature detectors (balanced mixers) 34 and 36 in the ultrasound signal processing systems illustrated in FIG. 2 and FIG. 4. The reflected wave received by the ultrasonic probe 22 and amplified by the amplifier 30 is applied to a quadrature demodulator circuit shown in FIG. 2 and FIG. 4. The signals derived from the transducer and input to the quadrature channels at 32 may be RF or IF; use of an RF beamformer signal is a preferred embodiment.

The oscillator 10 generates and applies a stable high frequency signal to the synchronizing circuit 12 which generates various output signals having a desired frequency. These output signals include a signal for causing repeated transmission of an ultrasonic pulse beam. This signal is applied through the transmitter circuit and the change-over circuit 16 to the ultrasound probe 22, and vibrators contained in the ultrasonic probe are excited to transmit an ultrasonic pulse beam toward and into the internal moving part of the living body under diagnosis. The internal moving part is, in this example, blood flow in the blood vessel 26. The sector 28 designates a region of scanning for the measurement of a Doppler blood flow image. A transmitter control circuit controls the ultrasonic pulse beam to transmit it at a predetermined scanning angle, direction and depth toward and into the sector 28. Pulses of the transmitted ultrasonic beam reflected from the various tissue interfaces in the body sector are received by the ultrasonic probe. The received echo signal, after being amplified by the amplifier 30, is then sent to the processing systems of FIG. 2 or FIG. 4 for further processing.

In order to estimate the Doppler shifts of the echoes received from blood cells, an ultrasound imaging system must transmit several (perhaps from 4 to 16) pulses at one location and then detect the variations in the phase of the echoes from pulse to pulse. Part of the conventional receiving process is shown in FIG. 2. A quadrature baseband detection process is used in which, in this example, the RF input signal 32 from the beamformer of FIG. 1 is applied to the quadrature detectors 34 and 36. Separately, the reference signal 16 from the oscillator 10 is applied to the quadrature detectors, with the reference signal sent to one quadrature detector 36 having its phase shifted 90° by a phase shift circuit 38, so that a 90° phase-shifted reference signal 40 is applied to the quadrature detector 36, together with the amplified echo signal 32 from the beamformer. The reference signal 16 applied to the quadrature detector 34, in the description to follow, is processed as part of an in-phase quadrature channel 42; and the phase-shifted signal 40 is processed in a separate quadrature channel 44 having system components similar to the in-phase channel 42. Therefore, the description to follow will suffice for both channels.

The outputs of the balanced mixers 34 and 36 are analog signals representing the product of the input signal and the input reference signal 16. The analog signal 46 is then amplified by a first amplifier 50, passed through a low-pass filter 52, and then amplified by a second amplifier 54. In the illustrated embodiment, the RF signal from the receiving beamformer is downconverted to baseband in the quadrature channels, using balanced mixers. A swept gain function was previously applied to the RF signal to compensate for ultrasound attenuation in tissue. The first amplifier 50 is a fixed gain stage. The low-pass filter 52 sets the detection bandwidth which can be between about 100 KHz and about 2.5 MHz.

The output signal 55 from the second amplifier 54 is then converted to a digital output signal 57 in an analog-to-digital converter 56 for processing in an MTI filter 58 to cancel stationary components of the received echo signals. Echoes from stationary, or nearly stationary structures in the body are much stronger than the echoes from blood cells, often by as much as 60 dB. The analog-to-digital converter 56 has a large dynamic range in order to keep the blood flow signals well above quantization noise and yet not clip on the stationary signals. The MTI filter is used to remove stationary, or slowly moving components after the signal has been converted to digital by the analog-to-digital converter 56. The MTI filter has a comb response with notches at the pulse repetition frequency and its harmonics. The MTI output signals 60 and 62 can be processed to extract the Doppler frequency information, at discrete ranges, either by an autocorrelation technique, or by a frequency domain processing such as by fast Fourier transform (FFT) techniques.

FIG. 3 illustrates components of a conventional system for further processing and imaging of the Doppler ultrasound blood flow information derived from the system shown in FIG. 2. The baseband information 60 and 62 is digitally processed in a velocity estimator 64 and a digital scan converter 66 and is then sent to a color processor 68 for further digital processing of the information. The results are converted by a digital-to-analog converter 70 into the three primary colors, red, green and blue, of the analog signals displayed on the CRT of a color TV 72. The output signals also can be converted into standard TV signals via an encoder 74 for connection to peripheral equipment such as a VCR 76.

The embodiment of FIG. 4 illustrates an analog feedback stationary signal canceller for ultrasonic flow imaging according to principles of this invention. The RF signal 32 from the receiving beamformer is downconverted to baseband in the quadrature channels, and the first amplifier 50 and low-pass filter 52 amplify the output signals and set the detection bandwidth which also may be between about 100 KHz and about 2.5 MHz. The canceller operates in two modes: stationary signal acquisition and then Doppler signal acquisition. These two modes occur in succession during each data acquisition interval. Electronic switched 80 and 82 and a demultiplexer 84 are shown in FIG. 4 in the stationary acquisition mode. An analog-to-digital converter 86, the addresses of a line buffer 88, and a digital-to-analog converter 90 are all clocked at a sample rate corresponding to the length of a flow image resolution cell. This sample rate is typically between about 200 KHz and about 5 MHz. In each flow measuring sequence, the ultrasound lines are directed toward a predetermined location in the body sector 28 under diagnosis. These take the form of pulse-echo sequences transmitted from and received by the probe 22 over a preselected time interval. The process is sequential and is then repeated, in succession, for the next flow image line. In the stationary acquisition mode, a single ultrasound line (pulse-echo) is first transmitted and received. This stationary signal is then stored in the line buffer 88, after which the system switches to the Doppler acquisition mode. The stored signal represents primarily stationary targets. The ultrasound line that was used to acquire the stationary signals is not used for flow estimation. On each of the succeeding pulses which are pulsed along the same line and toward the same target, the stored line samples are recalled from the line buffer 88, converted to analog form by a digital-to-analog converter 90, and subtracted from the new baseband signals 96. During the Doppler acquisition mode, the switch 80 is closed so that the analog signals 92 from the converter 90, representing the stationary signal components along the data line, are fed back to a summing junction 94 for subtracting these signals from the new baseband signals 96. An analog residue signal 98 output from the summing junction 94 contains the pulse-to-pulse variations which principally constitute the Doppler signal. During the Doppler acquisition mode, the switch 82 is closed so that the residue signals 99, which have then been boosted in gain by the amplifier 100, are then converted to digital in the analog-to-digital converter 86, shifted by the demultiplexer 84, and processed by an MTI filter 102. By boosting the gain of the Doppler signal, a large Doppler signal with improved dynamic range is then used in the following process of forming the velocity estimate.

The residue signal 99 contains the pulse-to-pulse variations which constitute the Doppler signal as well as some undesired components. Signals from tissue are completely cancelled, only if they are completely stationary. The MTI filter 102 removes the slowly varying component of the clutter. Error can also occur in a situation where there is a strong Doppler return but no stationary echo. In this instance, the worst case error amplitude is equal the peak Doppler signal amplitude. This error would appear as a DC component in the 4-to-16 sample sequence, and is also removable by the conventional type of MTI filter which follows the analog-to-digital converter 86. The errors limit the amount of gain that the amplifier 100 may have and, thus, limit the improvement in Doppler signal-to-clutter ratio. In the preferred embodiment, this gain can be altered as desired to suit different pulse frequencies, operating frequencies, and the like. The circuit can also be disabled by the user if the small reduction in image frame rate is undesirable and the Doppler-to-clutter ratio is not too unfavorable in the region of interest.

In a prior art system which has also used an analog feedback path around an analog-to-digital converter as part of a stationary canceller, the canceller is a first-order recursive filter which has notches at the pulse repetition frequency and its harmonics. This canceller updates its estimate of the stationary signal component on every pulse. The technique according to the present invention is different, in that it uses a single "snapshot" of the stationary signal to subtract from each of the 4-to-16 pulse-echo sequences that are acquired to estimate flow along one line. In flow imaging, only a few pulse-echo sequences are used to form estimates of the Doppler signals along each line. This invention takes advantage of the fact that, during the brief time interval used for the 4 to 16 pulses (a few milliseconds at the most), the quasi-stationary echoes from slowly moving tissue are in fact reasonably stable. Thus, the one-line sample of those echoes suffices to mostly cancel the "stationary" component. As a result, the system of this invention produces a higher ratio of Doppler signal-to-quantization noise with more accurate image information. The system also permits use of less expensive analog-to-digital converters with only a minimal decrease in image frame rate.

## Claims

1. An ultrasonic Doppler flow measuring and imaging system comprising:

   (a) ultrasonic wave transmitting and receiving means for sequentially transmitting ultrasonic waves toward and into a living body over a predetermined time interval and for receiving their reflected echo signals, wherein each received echo signal has a stationary component representative of reflection from essentially stationary tissue and a Doppler component representative of reflection from a moving part of the body;

   (b) means for processing the echo signals received during successive predetermined time intervals, the signal processing means including (i) means for subtracting a stationary component from the received echo signals to thereby extract from the echo signals a Doppler signal representative of a Doppler component of the received echo signals, and (ii) means for thereafter amplifying the Doppler signal to produce a succession of output signals representative of the amplified Doppler components of the reflected echo signals; and

   (c) means for processing the output signals to generate therefrom Doppler flow image data signals for use in imaging the moving part of the body.

2. Apparatus according to claim 1 in which the measuring and imaging system comprises an ultrasonic Doppler blood flow measuring system.

3. Apparatus according to claim 1 in which the echo signal processing means includes means for sampling multiple reflected echo signals during each time interval, and means for subtracting therefrom the stationary component based on fewer sampled echo signals than the Doppler component.

4. Apparatus according to claim 3 including, for each time interval, means for storing a reflected echo signal, means for sampling a plurality of other reflected echo signals, and means for subtracting the stored reflected echo signal from the sampled other echo signals to extract said Doppler signal.

5. Apparatus according to claim 4 including means for storing one reflected echo signal for each time interval.

6. Apparatus according to claim 4 in which the echo signal processing means includes means for passing the amplified Doppler signals through an analog-to-digital converter, and means for filtering the digital output in an MTI filter.

7. Apparatus according to claim 6 in which the filter output is received by a velocity estimator and display system.

8. Apparatus according to claim 7 in which the system comprises an ultrasonic Doppler blood flow measuring system and display.

9. Apparatus according to claim 8 in which the imaging system includes color imaging.

FIG-1

OSCILLATOR ~10

FREQUENCY DIVIDER
AND SYNC CIRCUIT ~12 ~16

~14

DRIVER ~18

~20

TRANSMIT-
RECEIVE ~30 H.F. AMP

24 ~22 ~32

28 ~26

FIG 3

COLOR T.V. ~72 VCR ~76

70 — D/A ~74 ENCODER

60 64 66 68

VELOCITY ESTIMATOR SCAN CONVERTER COLOR PROCESSOR ~68

62

7

*Fig 2*

RF FROM
BEAMFORMER

LOCAL
OSCILLATOR

~90°

~90°

INPHASE CHANNEL

QUADRATURE CHANNEL, SAME AS ABOVE

Prior Art

A1
LPF
A2
ADC
MTI
FILTER

TO VELOCITY
ESTIMATOR
AND DISPLAY
SUBSYSTEM

8

Fig 4

EP 0 430 093 A2